# EUROPEAN PATENT SPECIFICATION

(11) **EP 4 121 407 B1**
(45) Date of publication and mention of the grant of the patent: **06.03.2024**
(21) Application number: 21711585.6
(22) Date of filing: 17.03.2021
(51) Int. Cl.: C07C 51/15, C07C 51/43, C07C 57/04

(54) **CATALYTIC PROCESS FOR PREPARING AN ALPHA, BETA-ETHYLENICALLY UNSATURATED CARBOXYLIC ACID SALT**
KATALYTISCHES VERFAHREN ZUR HERSTELLUNG EINES ALPHA, BETA-ETHYLENISCH UNGESÄTTIGTEN CARBONSÄURESALZES
PROCÉDÉ CATALYTIQUE POUR LA PRÉPARATION D'UN SEL D'ACIDE CARBOXYLIQUE ALPHA, BETA-INSATURÉ

(30) Priority: 20.03.2020 EP 20164407
(43) Date of publication of application: 25.01.2023
(73) Proprietor: BASF SE, 67056 Ludwigshafen am Rhein (DE)
(72) Inventor: HUGUET SUBIELA, Nuria, 67056 Ludwigshafen (DE); WEISSKER, Wolf-Steffen, 67056 Ludwigshafen (DE)
(74) Representative: BASF IP Association
(86) International application number: PCT/EP2021/056750
(87) International publication number: WO 2021/185879

(56) References cited:
- WO-A1-2017/178282
- US-A1- 2013 172 616

## Description

The present invention relates to a catalytic process for preparing an α,β-ethylenically unsaturated carboxylic acid salt, wherein an alkene and carbon dioxide are contacted with a catalyst, N,N-dimethylformamide and an alkoxide. The unsaturated carboxylic acid salt is obtained as a solid which is mechanically separated from the reaction mixture and the alcohol by-product.

The direct addition of carbon dioxide onto alkenes to obtain α,β-ethylenically unsaturated carboxylic acid salts, for example onto ethene to give an acrylate, is of principal industrial interest due to the high availability and low price of the reactants. Research into overcoming the unattractive thermodynamic limitations and the unfavorable equilibrium, which at room temperature is virtually completely to the side of the reactants, has recently yielded a number of catalytic processes.

WO 2016/180775 A1 describes a process wherein a crude reaction product from the reaction of an alkene and CO₂ in the presence of a carboxylation catalyst and an alkoxide is contacted with a polar solvent such as water so as to obtain a first liquid phase in which the α,β-ethylenically unsaturated carboxylic acid salt is enriched, and a second liquid phase in which the carboxylation catalyst is enriched. The alcohol by-product is distilled off from the first liquid phase. The process achieves a catalytic turnover in a single stage while allowing for the separation of the carboxylic acid salt from the alcohol by-product. The known process imposes restrictions with regard to the usable reaction solvents since only those solvents can be employed that have limited miscibility with, e.g., water. In addition, although a second liquid phase in which the carboxylation catalyst is enriched separates from an aqueous phase in which the α,β-ethylenically unsaturated carboxylic acid salt is enriched, the second liquid phase entrains traces of water that tend to deactivate the carboxylation catalyst.

WO 2017/178282 A1 discloses the reaction of ethylene and carbon dioxide in the presence of a metal complex (Pd or Ni) and a secondary or tertiary alkoxide, such as e.g. sodium tert-butoxide. The reaction is carried out using solvents such as dibutylformamide, N-cyclohexylpyrrolidone or other nitrogenated solvents. The sodium acrylate product is isolated by separating the organic and aqueous phases, followed by evaporation of the solvent.

The problem underlying the present invention can be seen in providing a process for preparing an α,β-ethylenically unsaturated carboxylic acid salt that is not subject to the above limitations.

The problem is solved by providing a catalytic process for preparing an α,β-ethylenically unsaturated carboxylic acid salt, comprising
a) contacting an alkene and carbon dioxide with a carboxylation catalyst, an organic solvent, wherein the organic solvent is N,N-dimethylformamide, and an alkoxide having a secondary or tertiary carbon atom directly bound to an [O⁻] group, to obtain a crude reaction product comprising the α,β-ethylenically unsaturated carboxylic acid salt and an alcohol by-product which is the conjugate acid of the alkoxide,
b) allowing the α,β-ethylenically unsaturated carboxylic acid salt to precipitate out from the crude reaction product; and
c) subjecting at least part of the crude reaction product to a mechanical separation step while maintaining the alcohol by-product in liquid form to obtain a solid phase comprising the α,β ethylenically unsaturated carboxylic acid salt and a liquid phase comprising the carboxylation catalyst, the organic solvent and the alcohol by-product.

The α,β-ethylenically unsaturated carboxylic acid salt, due to its ionic nature, has a finite solubility in most organic solvents and precipitates out from the crude reaction product. It has been found that the process can be controlled in a way that other components of the reaction mixture, in particular the alcohol by-product, can be kept in a liquid and/or dissolved state so that the α,β-ethylenically unsaturated carboxylic acid salt can be easily separated by a mechanical separation operation.

Subjecting the crude reaction product to a mechanical separation step while maintaining the alcohol by-product in liquid form allows for obtaining the precipitated α,β-ethylenically unsaturated carboxylic acid salt in a solid phase of high purity. The mechanical separation step advantageously does not require the utilization of additional chemicals such as polar solvents, or the separation of non-miscible liquid phases from one another. For example, it is not necessary to use water in the process invention, which favorably reduces the number of by-products.

The solution behavior of the carboxylic acid salt and the alcohol by-product can be controlled by the choice of the organic solvent and/or the temperature of the process. Favorably, the alcohol by-product is essentially completely soluble in the organic solvent within the temperature range of the process steps a) to c).

Suitable alkenes are those of the following general formula 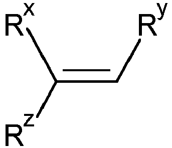 wherein
R^{x}, R^{y} and R^{z} are each independently hydrogen, C₁₋₁₂-alkyl, C₂₋₁₂-alkenyl, or R^{x} and R^{y} together with the carbon atoms to which they are bonded are a monoethylenically or diethylenically unsaturated, 5- to 8-membered carbocycle.

Suitable alkenes include ethene, propene, isobutene, butadiene, piperylene, 1-butene, 1-pentene, 1-hexene, 1-heptene, 1-octene, 1-nonene, 1-decene, 2-butene, cyclopentene, cyclohexene, cycloheptene, cyclooctene, cyclooctadiene, or styrene. The alkene to be used in the process according to the invention is generally gaseous or liquid under the reaction conditions.

In step a) the alkene partial pressure is, for example, in the range of 0.5 bar to 200 bar, preferably 1 bar to 100 bar, in particular 2 bar to 80 bar, further preferred 3 bar to 60 bar, most preferably 5 to 50 bar. All pressures indicated herein are absolute pressures.

In a preferred embodiment, the alkene is ethene. In this embodiment, the α,β-ethylenically unsaturated carboxylic acid salt obtained by the process of the invention is an acrylate.

In another embodiment, the alkene is piperylene. In this embodiment, the α,β-ethylenically unsaturated carboxylic acid salt obtained by the process of the invention is a sorbate.

The carbon dioxide for use in step a) of the process according to the invention can be used in gaseous, liquid or supercritical form. It is also possible to use CO₂-comprising gas mixtures available on the industrial scale, provided that they are substantially free of carbon monoxide.

The CO₂ and the alkene may also comprise inert gases such as nitrogen or noble gases. Advantageously, however, the content thereof is below 10 mol-%, based on the total amount of carbon dioxide and alkene in the process.

The molar ratio of CO₂ to alkene in step a) is generally in the range of 0.1 to 10 and preferably in the range of 0.5 to 5. The CO₂ and the alkene are generally fed into step a) in a molar ratio in the range of 0.1 to 10 and preferably in the range of 0.5 to 5 moles of CO₂ per mole of alkene.

The partial pressure of carbon dioxide in step a) is preferably maintained at above 1 bar. It is, for example, maintained at at least 2 bar, in particular at at least 4 bar, most preferably at at least 6 bar. The partial pressure of carbon dioxide in step a) is preferably maintained below 200 bar. It is, for example, maintained at at most 160 bar, in particular at at most 140 bar, most preferably at at most 120 bar. In step a) of the process according to the invention, the partial pressure of carbon dioxide is preferably maintained in a range of 1 to 200 bar, preferably 2 to 160 bar, in particular 4 to 140 bar, further preferred 6 to 120 bar, most preferably 10 to 100 bar.

The choice of organic solvent may be crucial for maintaining the alcohol by-product in liquid form while allowing the carboxylic acid salt to precipitate, along with the choice of temperature in step c) of the process.

Due to its ionic nature the α,β-ethylenically unsaturated carboxylic acid salt has a finite solubility in most organic solvents over a broad range of temperatures. Usually, the alcohol by-product precipitates from the organic solvent at lower temperatures than the α,β-ethylenically unsaturated carboxylic acid salt or is essentially completely soluble within the temperature range of the process steps.

The organic solvent of the process of the invention is N,N-dimethylformamide.

In another embodiment, the organic solvent may comprise a cosolvent, such as a polar, high-boiling cosolvent. The cosolvent may serve to maintain the solubility of the catalyst system during work-up. The cosolvent may be a glycol or a glycol derivative, or a mixture of such a compound with any of the compounds described as suitable above. Suitable organic cosolvents include glycols, glycol derivatives, and mixtures thereof. Examples of glycols include ethylene glycol (EG) and propylene glycol. Examples of glycol derivatives include polyalkylene glycols such as diethylene glycol (DEG) and triethylene glycol (TEG), di- or mono(C₁-C₄-alkyl ether) glycols such as ethylene glycol monomethyl or dimethyl ether, and di- or mono(C₁-C₄-alkyl ether) polyalkylene glycols such as diethylene glycol dimethyl ether, dipropylene glycol monomethyl ether and triethylene glycol dimethyl ether. Preferred glycol derivatives are end-capped compounds without free hydroxy groups, for example di(C₁-C₄-alkyl ether) glycols such as dimethyl ether and di(C₁-C₄-alkyl ether) polyalkylene glycols such as diethylene glycol dimethyl ether and triethylene glycol dimethyl ether.

In step a) of the process of the invention, the alkoxide having a secondary or tertiary carbon atom directly bound to an [O⁻] group is converted into an alcohol by-product, which is the conjugate acid of the alkoxide. The alkoxide is suitably at least partially soluble in the organic solvent. The alkoxide is consumed stoichiometrically.

The alkoxide used in the present process is an alkoxide having a secondary or tertiary carbon atom directly bound to an [0-] group. It is assumed that any undesired direct reaction of the alkoxide with CO₂ and in particular undesired half ester formation is more efficiently suppresses suppressed when the residue bound to the [O⁻] group is sterically demanding.

The term "alkoxide having a secondary or tertiary carbon atom directly bound to an [O⁻] group" refers to a compound comprising subunits of general formula (I)

-O-R (I)

wherein R is a hydrocarbyl residue which comprises a carbon atom that is bound to the oxygen atom of general formula (I), and the carbon atom bound to the oxygen atom of general formula (I) is a secondary carbon atom or a tertiary carbon atom. A secondary carbon atom is understood to mean a carbon atom directly bound to the oxygen atom of general formula (I) which has two carbon-carbon bonds and a carbon-hydrogen bond. A tertiary carbon atom is understood to mean a carbon atom directly bound to the oxygen atom of general formula (I) which has three carbon-carbon bonds.

Tertiary moieties are generally more sterically demanding than secondary moieties. Thus, the alkoxide having a secondary or tertiary carbon atom directly bound to the [O⁻] group is preferably an alkoxide having a tertiary carbon atom directly bound to the [O⁻] group ("a tertiary alkoxide").

The hydrocarbyl residue R may be acyclic, cyclic or comprise cyclic substructures. R is preferably acyclic. The hydrocarbyl residue R may be saturated or unsaturated. Hydrocarbyl residues R that are saturated comprise only single bonds, whereas hydrocarbyl residues R that are unsaturated comprise at least one double bond. The hydrocarbyl residue R is preferably saturated.

Any hydrogen atom of R may be substituted by one or multiple non-interfering substituents. A non-interfering substituent is a substituent that is inert under the conditions of the process according to the invention, i.e. a substituent that does not react with any compound or intermediate being in contact with the alkoxide or with the conjugate acid of the alkoxide in the process according to the invention. Suitable substituents include -F, -Cl, and -O-C₁-C₆-alkyl. R is preferably unsubstituted, which means that R consists solely of carbon and hydrogen atoms.

The alkoxide may be derived from an alcohol with multiple hydroxy groups, i.e. a polyol, such as a diol like ethylene glycol, diethylene glycol, propane diol or butane diol. It is in this case understood that the term "alkoxide having a secondary or tertiary carbon atom directly bound to an [O⁻] group" means that to every [O⁻] group, a secondary or tertiary carbon atom is directly bound.

In a preferred process, the alkoxide is derived from an alcohol that does not decompose when being distilled. The alkoxide may thus be selected based on the decomposition temperature of its conjugate acid, i.e. of the conjugate alcohol. It is generally preferable that the decomposition temperature of the conjugate alcohol is significantly higher than its boiling temperature at 1 bar. The decomposition temperature of the conjugate acid of the alkoxide, i.e. of the conjugate alcohol, is preferably at least 10 °C, in particular at least 20 °C, for example 30 °C to 200 °C higher than its boiling temperature at 1 bar. It is preferable that the boiling temperature at 1 bar is not too high, so as to provide an energetically favorable process.

This difference between decomposition temperature and boiling temperature may be established by choosing the alkoxide based on the molecular weight of its conjugate acid, i.e. of the conjugate alcohol. Low molecular weight compounds tend to have low boiling temperatures. The molecular weight of the conjugate acid of the alkoxide is, for example, in the range of 55 to 200 g/mol, preferably 70 to 180 g/mol, in particular 95 to 180 g/mol, most preferably 100 to 160 g/mol.

In one embodiment, the boiling point of the conjugate acid of the alkoxide, i.e. of the conjugate alcohol, is suitably between the boiling point of methanol and the boiling point of the organic solvent. In another embodiment, the boiling point of the conjugate acid of the alkoxide, i.e. of the conjugate alcohol, is suitably higher than the boiling point of both the organic solvent and of methanol.

In preferred embodiments, the difference between the boiling point of the organic solvent and the boiling point of the conjugate alcohol at 1 bar absolute is at least 5 °C, preferably at least 8 °C and most preferably at least 25 °C.

In a preferred process, the alkoxide having a secondary or tertiary carbon atom directly bound to an [O⁻] group comprises subunits of general formula (II)

-O-CR¹(R²)₂ (II)

wherein
R¹ is H or R², and
each R² is independently selected from C₁-C₁₀-hydrocarbyl or any two or three R² together with the secondary or tertiary carbon atom to which they are bonded are one or multiple 3- to 8-membered carbocycles.

Each R² is, for example, independently selected from C₁-C₁₀-alkyl, C₃-C₁₀-cycloalkyl, and C₆-C₁₀-aryl. Preferably, each R² is independently selected from C₁-C₆-alkyl.

Preferably, the alkoxide having a secondary or tertiary carbon atom directly bound to an [O⁻] group comprises subunits selected from -O-tert-butyl, -O-isopropyl, -O-sec-butyl, - O-cyclopropyl, and -O-((1-methyl)-cyclopropyl), -O-cyclohexyl, and -O-((1-methyl)-cyclohexyl)).

Preferably, the alkoxide is acyclic and has a tertiary carbon atom directly bound to an [O⁻] group. The alkoxide having a secondary or tertiary carbon atom directly bound to an [O⁻] group is, for example, selected from alkali metal alkoxides and alkaline earth metal alkoxides. Alkali metal and in particular sodium alkoxides are preferred, especially sodium alkoxides having a tertiary carbon atom directly bound to an [O⁻] group.

Particularly preferred alkoxides having a secondary or tertiary carbon atom directly bound to an [O⁻] comprise 3 to 15 carbon atoms. The most preferred alkoxides having a secondary or tertiary carbon atom directly bound to an [0-] comprise 5 to 10 carbon atoms.

Examples of preferred sodium alkoxides having a secondary or tertiary carbon atom directly bound to an [O⁻] include:
sodium isopropoxide (sodium propan-2-olate),
sodium tert-butoxide,
sodium cyclopentanolate,
sodium cyclohexanolate,
sodium cycloheptanolate,
sodium butan-2-olate,
sodium 3-methylbutan-2-olate,
sodium 4-methylpentan-2-olate,
sodium pentan-3-olate,
sodium 1-methoxypropan-2-olate,
sodium 1-methylcyclopentan-1-olate,
sodium 1-methylcyclohexan-1-olate,
sodium 2-phenylpropan-2-olate,
sodium 3-methylheptan-3-olate,
sodium 3-methylhexan-3-olate,
sodium 2-methylhexan-2-olate
sodium 2-methylbutan-2-olate,
sodium 3-ethylpentan-3-olate,
sodium 2-methylpentan-2-olate,
sodium 3-methylpentan-3-olate,
sodium 3,7-dimethyloctan-3-olate, and
sodium 2,3-dimethyl-2-butanolate.

Sodium tert-butoxide, sodium 3-methylpentan-3-olate, sodium 3-ethylpentan-3-olate and sodium 3,7-dimethyloctan-3-olate are particularly preferred alkoxides, especially sodium 3-methylpentan-3-olate, sodium 3-ethylpentan-3-olate and sodium 3,7-dimethyloctan-3-olate.

The alkoxide can, for example, be added in solid form, as a neat liquid (in cases where the alkoxide is in liquid form at room temperature), or as a solution.

The process comprises contacting an alkene and carbon dioxide with a carboxylation catalyst. The carboxylation catalyst is preferably a transition metal complex.

In step a), the carboxylation catalyst is preferably present at 0.1 to 20,000 ppm by weight, preferably 1 to 1,000 ppm by weight, in particular 5 to 500 ppm by weight of the transition metal, based on the total weight of the reaction mixture.

The term "transition metal complex" used herein comprises, in a generic manner, all transition metal complexes through which the catalytic cycle is supposed to pass, i.e. transition metal-alkene complexes, metallalactones and adducts wherein the α,β-ethylenically unsaturated carboxylic acid salt coordinates to the transition metal.

In general, the transition metal complex comprises, as the active metal, at least one element of groups 4 (preferably Ti, Zr), 6 (preferably Cr, Mo, W), 7 (preferably Re), 8 (preferably Fe, Ru), 9 (preferably Co, Rh) and 10 (preferably Ni, Pd, Pt) of the Periodic Table of the Elements. Preference is given to nickel and palladium. Most preferably, the transition metal complex is a palladium complex.

The role of the active metal consists in the activation of CO₂ and the alkene in order to form a C-C bond between CO₂ and the alkene. It is assumed that a metallalactone is formed within the catalytic cycle from the alkene, carbon dioxide and the transition metal complex. The expression "metallalactone" denotes, according to the exchange nomenclature ("a" nomenclature), a lactone (γ-lactone) in which a carbon atom has been exchanged for a metal atom. The expression "metallalactone" should be interpreted broadly and may comprise compounds with structures similar to the Hoberg complex, or related compounds of oligomeric or polymeric structure. The expression shall comprise isolable compounds and (unstable) intermediates.

The metallalactone can be illustrated by the following general formula 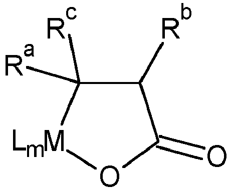 in which
- M: is the transition metal,
- L: is a ligand,
- m: is 1 or 2, and
- R^{a}, R^{b} and R^{c}: are each independently hydrogen, C₁₋₁₂-alkyl, C₂₋₁₂-alkenyl, or R^{a} and R^{b} together with the carbon atoms to which they are bonded are a saturated or mono- or diethylenically unsaturated, 5- to 8-membered carbocycle.

It is assumed that the alkoxide deprotonates the metallalactone at the carbon atom α to the carbonyl group.

Preferably, the transition metal complex comprises a ligand that coordinates to the transition metal via at least one ligand atom selected from P, N, O, and C. The ligand preferably comprises at least one phosphorus atom which coordinates to the transition metal. The ligand may be monodentate or polydentate, for example bidentate. In general, two monodentate ligands or one bidentate ligand coordinate to the transition metal.

The polydentate, e.g. bidentate, ligand may coordinate to the transition metal to form a four-, five-, six-, seven-, or eight-membered ring, i.e. the transition metal, the atoms which coordinate to the transition metal, and the atoms of the shortest chain which connects the atoms coordinating to the transition metal, together form a four-, five-, six-, seven-, or eight-membered ring. Ligands that coordinate to the transition metal, e.g., nickel or palladium, to form a five-, six-, or seven-membered ring are preferred.

Alternatively, the atoms which coordinate to the transition metal may be directly bound to carbon atoms of two cyclopentadienyl ligands bound to a second metal, e.g., iron.

At least one residue is preferably bound via a secondary or tertiary carbon atom to a transition metal coordinating phosphorus atom. More particularly, at least two residues are preferably bound to the phosphorus atom via a secondary or tertiary carbon atom. Preferred residues bound to the phosphorus atom via a secondary or tertiary carbon atom are adamantyl, tert-butyl, sec-butyl, isopropyl, cyclohexyl, and cyclopentyl.

The ligand is preferably a bidentate P,X ligand in which X is selected from the group consisting of P, N, O, and carbene, in particular a bidentate P,P ligand. The P and X atoms are, for example, separated by a bivalent linker that comprises 2 to 4 bridging atoms. The linker is preferably linked to the P atom by a single bond and linked to the X atom by a single bond and comprises 2 to 4 bridging atoms linked by single bonds.

The bidentate P,X ligand, in particular the bidentate P,P ligand, may be structurally constrained or unconstrained. It is preferably structurally unconstrained.

In the structurally constrained bidentate P,X ligand, in particular in the bidentate P,P ligand, the bridging atoms may be part of at least one cyclic substructure, in particular of at least one 5- to 7-membered cyclic substructure. In preferred bidentate P,P ligands, wherein the bridging atoms are part of at least one 5- to 7-membered cyclic substructure, each bridging atom directly linked to a P atom, together with the P atom to which it is linked, is part of a 5- to 7-membered cyclic substructure; or two neighboring bridging atoms are part of a 5- to 7-membered cyclic substructure.

Preferred structurally constrained bidentate P,P ligands are ligands of formula (Illa) 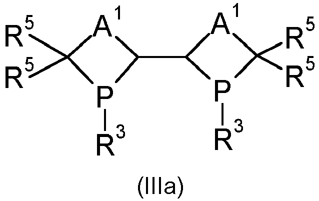 wherein
- R³: is independently selected from CHR⁴₂, CR⁴₃, C₃-C₁₀-cycloalkyl, and optionally alkylated aryl having 6 to 18 carbon atoms,
- R⁴: is independently selected from C₁-C₄-alkyl, preferably linear C₁-C₄-alkyl,
- A¹: together with the carbon atoms to which it is bound and the interjacent phosphorous atom forms a 5- to 7-membered cyclic substructure, and
- R⁵: is independently selected from hydrogen, C₁-C₁₂-alkyl, C₃-C₁₂-cycloalkyl, C₃-C₁₂-heterocycloalkyl, C₆-C₁₄-aryl, C₆-C₁₄-heteroaryl, C₁-C₁₂-alkoxy, C₃-C₁₂-cycloalkoxy, C₃-C₁₂-heterocycloalkoxy, C₆-C₁₄-aryloxy, and C₆-C₁₄-heteroaryloxy.

A¹ is preferably selected from -(CR^{5'}₂)ⱼ- and -(CR⁶=CR⁶)ₖ- with both R⁶ being on the same side of the double bond, wherein R^{5'} is independently selected from H, C₁-C₃-alkyl, and -O-C₁-C₃-alkyl, R⁶ is selected from H and C₁-C₃-alkyl, or at least two R⁶ constitute a bridge of one of the formulae: 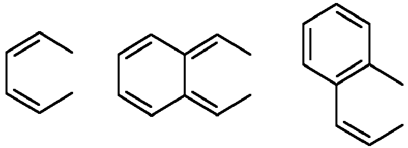 j is 2 or 3, and k is 1 or 2.

R³ is preferably independently selected from CHR⁴₂, CR⁴₃, and C₃-C₈-cycloalkyl, most preferably CR⁴₃.

R⁴ is preferably methyl.

R⁵ is preferably H.

A¹ is preferably selected from ethylene, ethenylene, 1,2-phenylene, 1,2-naphthylene, 2,3-naphthylene, and the following formulae:

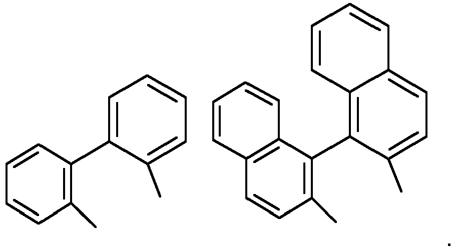

Preferred structurally constrained bidentate P,P ligands are ligands of formula (Illb) 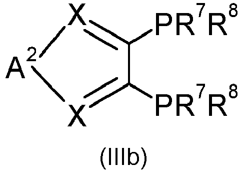 wherein
- R⁷: is independently selected from linear C₁-C₄-alkyl,
- R⁸: is independently selected from CHR⁷₂, CR⁷₃, C₃-C₁₀-cycloalkyl, and optionally alkylated aryl having 6 to 18 carbon atoms,
- X: is independently selected from C-H, C-CH₃, and N, and
- A²: together with the moieties X to which it is bound and the interjacent carbon atoms forms a 5- to 7-membered cyclic substructure.

R⁷ is preferably independently selected from C₁-C₆-alkyl and C₃-C₇-cycloalkyl and R⁸ is CR¹⁰₃.

R⁷ may, for example, be independently selected from linear C₁-C₄-alkyl, in particular from linear C₁-C₂-alkyl.

R⁸ is preferably independently selected from CHR⁷₂, CR⁷₃, and C₃-C₈-cycloalkyl.

A² is preferably a -CH=CH- bridge.

X is preferably CH.

Preferred structurally constrained bidentate P,P ligands are ligands of formula (Illc) 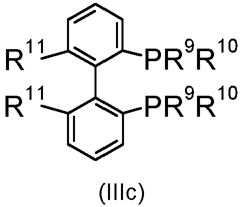 wherein
- R⁹: and R¹⁰ are independently selected from C₃-C₁₀-cycloalkyl, e.g, C₅-C₇-cycloalkyl, and
- R¹¹: is H, O-C₁-C₆-alkyl, or both R¹¹ together constitute a -CH=CH- bridge.

R¹¹ is preferably H or OCH₃ and most preferably H.

In the preferred structurally unconstrained bidentate P,X ligand, e.g., bidentate P,P ligand, each of the bridging atoms is unbranched and none of the bridging atoms is part of any cyclic substructure apart from the ring including the transition metal.

In a particularly preferred bidentate P,P ligand, each of the bridging atoms is unbranched and none of the bridging atoms is part of any cyclic substructure apart from the ring including the transition metal. In a particularly preferred bidentate P,P ligand, the P atoms are separated by a linker that comprises 2 bridging carbon atoms, each P atom being linked to the linker and to two secondary carbon bound residues by single bonds, and the bridging carbon atoms being linked by a single bond. The expression "secondary carbon bound residue" refers to a residue that is bound to the P atom via a secondary carbon atom that is comprised by the residue. Each of the four secondary carbon bound residues may be the same or different and preferably selected from secondary C₃-C₂₀-hydrocarbyl residues wherein any hydrogen atom comprised by the secondary C₃-C₂₀-hydrocarbyl residues may be substituted by one or multiple non-interfering substituents. A non-interfering substituent is a substituent that is inert under the conditions of the processes according to the invention, i.e. a substituent that does not react with any compound or intermediate being in contact with the ligand in the processes according to the invention. Suitable non-interfering substituents include, for example, O-C₁-C₆-alkyl. The secondary C₃-C₂₀-hydrocarbyl residues are preferably unsubstituted which means that they consist of hydrogen and carbon atoms. Preferred unsubstituted secondary C₃-C₂₀-hydrocarbyl residues are 2-propyl, 2-butyl, 2-pentyl, 3-pentyl, cyclopentyl, cyclohexyl, and cycloheptyl. The preferred linker is -CH₂-CH₂-.

Preferred structurally unconstrained bidentate P,P ligands are ligands of formula (IIId)

R¹²R¹³P-(CR¹⁴R¹⁵)ₑ-PR¹²R¹³ (IIId)

wherein
R¹² and R¹³ are independently an unbranched or branched, acyclic or cyclic, aliphatic residue having 1 to 20 carbon atoms, where individual carbon atoms may independently be replaced by a hetero group selected from the group of -O- and >N-, individual hydrogen atoms may independently be replaced by Cl, or F, and any two residues bound to the same phosphorous atom may be covalently bound to one another,
e is 1, 2, 3, 4, or 5, preferably 2, 3, or 4,
R¹⁴ is independently selected from H, C₁-C₈-alkyl, C₁-C₈-alkoxy, C₃-C₁₀-cycloalkyl, C₃-C₁₀-cycloalkoxy, C₆-C₁₀-aryl, and C₆-C₁₀-aryloxy, or two vicinal R¹⁴ residues, together with the carbon atoms to which they are attached, form a 5- to 7-membered carbocycle, which can be aromatic, partially unsaturated, or saturated; and
R¹⁵ is independently selected from H, C₁-C₈-alkyl, C₃-C₁₀-cycloalkyl, and C₆-C₁₀-aryl.

Preferably, (CR¹⁴R¹⁵)ₑ is -CH₂-CH₂-, -CH₂-CH₂-CH₂-, or -CH₂-CH₂-CH₂-CH₂-.

R¹² and R¹³ are preferably independently C₁-C₂₀-alkyl, or C₃-C₂₀-cycloalkyl, wherein C₁-C₂₀-alkyl is unsubstituted or substituted with 1, 2, 3, or 4 substituents independently selected from Cl, F, and C₁-C₄-alkoxy and wherein C₃-C₂₀-cycloalkyl is unsubstituted or substituted with 1, 2, 3, or 4 substituents independently selected from Cl, F, C₁-C₈-alkyl and C₁-C₄-alkoxy.

R¹² and R¹³ are most preferably independently methyl, ethyl, 1-propyl, 2-propyl, 1-butyl, 2-butyl, tert-butyl, 1-(2-methyl)propyl, 1-pentyl, 1-(2-methyl)pentyl, 1-hexyl, 1-(2-ethyl)hexyl, 1-heptyl, 1-(2-propyl)heptyl, 1-octyl, 1-nonyl, 1-decyl, 1-undecyl, 1-dodecyl, adamantyl, cyclopentyl, methylcyclopentyl, cyclohexyl, methylcyclohexyl, cycloheptyl, cyclooctyl, or norbornyl, in particular independently 2-propyl, 2-butyl, tert-butyl, adamantyl, cyclopentyl, methylcyclopentyl, cyclohexyl, methylcyclohexyl, cycloheptyl, cyclooctyl, or norbornyl.

Particularly preferred bidentate P,P ligands are ligands of formula (llld-1)

R¹²R¹³P-(CR¹⁴H)ₑ-PR¹²R¹³ (IIId-1)

wherein
- R¹² and R¹³: are each independently an unbranched or branched, acyclic or cyclic aliphatic residue having 1 to 20 carbon atoms,
- e: is 2, 3, or 4, and
- R¹⁴: is independently selected from H, C₁-C₈-alkyl, C₁-C₈-alkoxy, C₃-C₁₀-cycloalkyl, C₃-C₁₀-cycloalkoxy, C₆-C₁₀-aryl, and C₆-C₁₀-aryloxy, or two vicinal R¹⁴ residues, together with the carbon atoms to which they are attached, form a 5- to 7-membered carbocycle, which can be aromatic, partially unsaturated, or saturated. Preferably, R¹⁴ is H.

In a particularly preferred process according to the invention, the ligand is selected from 1,2-bis(dicyclohexylphosphino)ethane, 1,3-bis(dicyclohexylphosphino)propane, 1,4-bis(dicyclohexylphosphino)butane, 2,3-bis(dicyclohexylphosphino)butane, 1,2-bis(dicyclopentylphosphino)ethane, 1,3-bis(dicyclopentylphosphino)propane, 1,4-bis(dicyclopentylphosphino)butane, 1,2-bis(dicycloheptylphosphino)ethane, 1,3-bis(dicycloheptylphosphino)propane, 1,4-bis(dicycloheptylphosphino)butane, 1,2-bis(diisopropylphosphino)ethane, 1,3-bis(diisopropylphosphino)propane, 1,4-bis(diisopropylphosphino)butane, 1,2-bis(di-sec-butylphosphino)ethane, 1,3-bis(di-sec-butylphosphino)propane, 1,4-bis(di-sec-butyl phosphino)butane, 1,2-bis(dodecylphosphino)ethane, 1,3-bis(dodecylphosphino)propane, 1,4-bis(dodecylphosphino)butane, 1,2-bis(decylphosphino)ethane, 1,3-bis(decylphosphino)propane, 1,4-bis(decylphosphino)butane, 1,2-bis(tetradecylphosphino)ethane, 1,3-bis(tetradecylphosphino)propane, 1,4-bis(tetradecylphosphino)butane, 1,2-bis(hexadecylphosphino)ethane, 1,3-bis(hexadecylphosphino)propane, 1,4-bis(hexadecylphosphino)butane, 1,2-bis(di-tert-butylphosphino)ethane, 1,3-bis(di-tert-butylphosphino)propane, 1,4-bis(di-tert-butylphosphino)butane, 1,2-bis(dicyclohexylphosphino)cyclohexane and 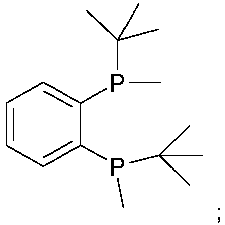 preferably from 1,2-bis(dicyclohexylphosphino)ethane, 2,3-bis(dicyclohexylphosphino)butane, 1,2-bis(diisopropylphosphino)ethane, 1,2-bis(dodecylphosphino)ethane, 1,2-bis(di-tert-butylphosphino)ethane, 1,2-bis(dicyclopentylphosphino)ethane, 1,2-bis(dicyclohexylphosphino)cyclohexane and

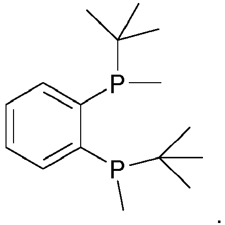

Suitable ligands are, for example, bidentate and multidentate ligands that comprise one or two coordinating phosphorous atoms and an additional carbon atom or hetero atom that is bound to the transition metal. Preferably, a 5-membered ring is formed, when the additional carbon atom or hetero atom binds to the transition metal, as for example with (diphenylphosphino)acetate known from the SHOP-Process or with 2-(dimethylphosphino)-N,N-dimethylethanamine. Specific bidentate ligands are ligands of formula (IIIg) 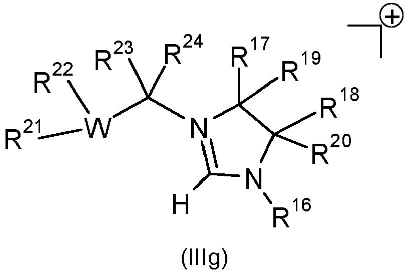 wherein
- W: is phosphorous (P) or phosphite (P=O),
- R¹⁶: is independently an unbranched or branched, acyclic or cyclic, aliphatic, araliphatic or aromatic residue having 1 to 16 carbon atoms, where individual carbon atoms may independently be replaced by a hetero group selected from the group of -O- and >N-, and where individual hydrogen atoms may independently be replaced by Cl or F,
- R¹⁷ and R¹⁸: are independently an unbranched or branched, acyclic or cyclic, aliphatic, araliphatic or aromatic residue having 1 to 16 carbon atoms, where individual carbon atoms may independently be replaced by a hetero group selected from the group of -O- and >N-, individual hydrogen atoms may independently be replaced by Cl, Br, I, or F, and both residues may be covalently bound to one another,
- R¹⁹ and R²⁰: together are a chemical bond, or as defined for R¹⁷ and R¹⁸,
- R²¹ and R²²: are as defined for R¹⁷ and R¹⁸, and
- R²³ and R²⁴: are as defined for R¹⁷ and R¹⁸.

Preferably R¹⁷, R¹⁸, R¹⁹ and R²⁰ are independently hydrogen, C₁-C₁₂-alkyl, or C₁-C₁₄-aryl; or R¹⁷ and R¹⁸ are independently hydrogen, C₁-C₁₂-alkyl, or C₁-C₁₄-aryl, and R¹⁹ and R²⁰ together are a chemical bond; or R¹⁷ and R¹⁸ are independently hydrogen, or methyl, and R¹⁹ and R²⁰ together are a C₃-C₁₀-alkane-1,3-diyl, C₃-C₁₀-alkane-1,4-diyl, or C₃-C₁₀-alkane-1,3-diyl bridge; or R¹⁹ and R²⁰ together are a chemical bond, and R¹⁷, and R¹⁸, together with the carbon atoms to which they are bound, are part of a monocyclic or bicyclic aromatic ring system.

R¹⁶, R²¹ and R²² are preferably independently C₁-C₁₂-alkyl, C₃-C₁₂-cycloalkyl, or C₃-C₁₄-aryl, wherein C₃-C₁₂-cycloalkyl and C₃-C₁₄-aryl are unsubstituted or substituted with 1, 2, 3, or 4 substituents independently selected from Cl, F, C₁-C₈-alkyl and C₁-C₄-alkoxy.

R¹⁶, R²¹ and R²² are most preferably independently methyl, ethyl, 1-propyl, 2-propyl, 1-butyl, 2-butyl, tert-butyl, 1-(2-methyl)propyl, 2-(2-methyl)propyl, 1-pentyl, 1-(2-methyl)pentyl, 1-hexyl, 1-(2-ethyl)hexyl, 1-heptyl, 1-(2-propyl)heptyl, 1-octyl, 1-nonyl, 1-decyl, 1-undecyl, 1-dodecyl, adamantyl, cyclopentyl, methylcyclopentyl, cyclohexyl, methylcyclohexyl, cycloheptyl, cyclooctyl, norbornyl, phenyl, napthyl, tolyl, xylyl, chlorophenyl or anisyl.

The ligand may also be a bidentate or multidentate ligand that comprises one or two coordinating nitrogen atoms and an additional carbon atom that is bound to the transition metal. Preferably, a 5-membered ring is formed, when the additional carbon atom binds to the transition metal, as for example with 2-phenylpyridine or 6-phenyl-2,2'-bipyridine.

Suitable tridentate ligands are, for example, ligands of formula (IIIh)

R¹²R¹³P-(CR¹⁴R¹⁵)_{f}-PR¹²-(CR¹⁴R¹⁵)_{g}-PR¹²R¹³ (IIIh)

wherein
- R¹², R¹³, R¹⁴, and R¹⁵: are each as already defined, and
- f and g: are independently 1, 2, 3, 4, or 5, preferably 2, 3, or 4

Exemplary tridentate ligands are ((methylphosphinediyl)bis-(methylene))-bis(dimethylphosphine), ((ethylphosphindiyl)bis(methylene))bis(diethyl-phosphine), and ((methylphosphinediyl)bis(methylene))bis(diphenylphosphine).

In addition to the above-described ligands, the transition metal complex may also have at least one further ligand selected from the alkoxide, the alcohol by-product which is the conjugate acid of the alkoxide, halides, amines, amides, oxides, phosphides, carboxylates, acetylacetonate, aryl- or alkylsulfonates, hydride, CO, olefins, dienes, cycloolefins, nitriles, aromatics and heteroaromatics, ethers, PF₃, phospholes, and mono-, di- and polydentate phosphinite, phosphonite, phosphoramidite and phosphite ligands.

Any of these further ligands can be displaced when the alkene and carbon dioxide are reacted.

The transition metal complex, e.g., palladium or nickel complex, may for example be obtained from the ligand and the transition metal, e.g., palladium or nickel, or from the ligand and a transition metal source, e.g., palladium or nickel source, comprising the transition metal, e.g., palladium or nickel, at oxidation state 0. Alternatively, the transition metal complex may for example be obtained by reducing a salt of the transition metal with a reducing agent, e.g., H₂, Mg, Na or Zn.

Palladium sources include, for example, PdL₂, PdL₄, LPdX₂, L₂PdX₂, L₂Pd₂X₂, LPd₂X₄, Pd₃X₆, L₃Pd₂, L₂Pd₂,
wherein
- X: is selected from halide, pseudohalide, carboxylate, alkoxide, carbonate, sulfate, nitrate, hydroxide, acetylacetonate, cyclopentadiene, alkyl, and aryl, and
- L: is a neutral ligand selected from phosphine, amine, olefin, carbonyl and nitrile,
and the corresponding adducts with solvents such as ethers, DMSO, or water.

The palladium sources and salts are preferably selected from [Pd₂(allyl)₂(Cl)₂], [Pd₂(methallyl)₂(Cl)₂] [Pd(dba)₂], [Pd₂(dba)₃], PdCl₂, PdBr₂, PdI₂, Pd(NO₃)₂, PdSO₄ [Pd(OAc)₂], [Pd(PtBu₃)₂], [Pd(PCy₃)₂], [Pd(PoTolyl₃)₂], [Pd(PPh₃)₄], [Pd(COD)(CI)(Me)], [Pd(Phen)(OAc)₂], [Pd₂(PtBu₃)₂(Br)₂], [Pd(C₆H₅CN)₂(Cl)₂], [Pd(PCy₃)₂(Cl)₂], [Pd(PPh₃)₂(Cl)₂], [Pd(norbornadiene)(Cl)₂], [Pd(TMEDA)(Cl)₂], [Pd(TMEDA)(CH₃)₂], [Pd₃(OAc)₆], [Pd(CF₃COO)₂], [Pd(acetylacetonate)₂], [Pd(COD)(Cl)₂], and [Pd(allyl)(Cp)].

Nickel sources and salts include, for example, NiL₂, NiL₄, LNiX₂, L₂NiX₂, L₂Ni₂X₂ wherein X and L are as defined above and the corresponding adducts with solvents such as ethers, DMSO, or water.

The nickel sources and salts are preferably selected from [Ni(COD)₂], NiF₂, NiCl₂, NiBr₂, Nil₂, [Ni(OAc)₂], [Ni(acetylacetonate)₂], [Ni(Ph₃P)₂(Cl)₂], [Ni((PPh₂)₂Fc)(Cl)₂], [Ni₂(methallyl)₂(Cl)₂], [Ni₂(allyl)₂(Cl)₂], [Ni(CO)₄], [Ni(PPh₃)₂(CO)₂], [Ni(NO₃)₂], [Ni(OH)₂], [Ni(PPh₃)₄], [Ni(CF₃COO)₂], [Ni(SO₄)], [Ni(2-ethylhexanoate)₂], [Ni(P(OPh)₃)₄], [Ni(C₇H₁₅COO)₂], [Ni(Cp)₂], [Ni(PCy₃)₂(Cl)₂], [Ni(PMe₃)₂(Cl)₂], [Ni(PBu₃)₂(Br)₂], and [Ni(dppe)(Cl)₂].

The following abbreviations are used in the above palladium and nickel sources and salts:
- dba:: dibenzylideneacetone
- Cy:: Cyclohexyl
- COD:: 1,5-Cyclooctadiene
- Phen:: Phenanthroline
- TMEDA:: N,N,N',N'-tetramethylethylenediamine
- Fc:: Ferrocenyl
- Cp:: Cyclopentadienyl

Many of the above palladium and nickel sources and salts are commercially available.

In preferred embodiments, the transition metal complex is present in homogeneous solution in the reaction medium in the form of complex-type compounds.

It may happen that part of the carboxylation catalyst is deactivated by oxidation of the active metal, e.g., nickel or palladium. The deactivation reduces the overall efficiency of the process. In this case, a reducing agent can be added. Apparently, the reducing agent reactivates the deactivated carboxylation catalyst by reduction of the oxidized active metal. Any reducing agent which is capable of reducing the deactivated carboxylation catalyst is suitable as the reducing agent. Preferable reducing agents are H₂, Mg, Na and Zn, or Phosphines.

In step a) of the process, a crude reaction product is obtained which comprises the α,β-ethylenically unsaturated carboxylic acid salt and the conjugate acid of the alkoxide.

Step a) may, for example, be performed at temperatures in the range of 20 to 250 °C, in particular 40 to 200 °C, preferably 50 to 190 °C, further preferably 60 to 180 °C, most preferably 70 to 180 °C. Step a) may, for example, be performed at a total pressure in the range of 1 to 300 bar, preferably 3 to 200 bar, in particular 5 to 150 bar.

Step a) is preferably carried out in a reactor which is suitable for gas/liquid reactions or liquid/liquid reactions at the given temperature and the given pressure. Suitable standard reactors for gas-liquid reaction systems are specified, for example, in K. D. Henkel, "Reactor Types and Their Industrial Application", in Ullmann's Encyclopedia of Industrial Chemistry 2005, Wiley VCH Verlag GmbH & Co KGaA, DOl: 10.1002/14356007.b04_087, chapter 3.3 "Reactors for gas-liquid reactions". Examples include stirred tank reactors, tubular reactors or bubble columns.

Step a) of the process according to the invention may be performed continuously or discontinuously. Step a) is preferably performed continuously. Step a) can also be carried out in several sequenced reactors. In this embodiment, it is preferable that step a) is carried out in a backmixed reactor, such as a stirred-tank reactor or jet loop reactor, and subsequently in a tubular reactor.

In a discontinuous step a), the ligand, the carboxylation catalyst which may for example be in the form of the transition metal source, the alkoxide, the organic solvent, carbon dioxide and the alkene are given into the reactor. Preferably, gaseous carbon dioxide and gaseous alkene are passed into the reactor at the desired pressure. After the reaction has slowed down, the pressure may be reduced.

In order to achieve good mixing of the alkene, carbon dioxide, the carboxylation catalyst, the organic solvent and the alkoxide, suitable apparatuses can be used in step a). Such apparatuses may be mechanical stirrer apparatuses with one or more stirrers, with or without baffles, packed or non-packed bubble columns, packed or non-packed flow tubes with or without static mixers, jet loop reactors or other useful apparatuses known to those skilled in the art for such a process step. The optional use of baffles and delay structures is explicitly included in the process according to the invention.

CO₂, alkene and the alkoxide can be fed into step a) either together or spatially separately. Such a spatial separation can be accomplished, for example in a stirred tank, in a simple manner by means of two or more separate inlets. When more than one tank is used, for example, there may be different media charges in different tanks. Separation of the addition of the CO₂ and alkene reactants in terms of time is also possible in step a) of the process according to the invention. Such a time separation can be accomplished, for example, in a stirred tank by staggering the charging with the reactants. In the case of use of flow tubes or apparatus of a similar kind, such charging can be effected, for example, at different sites in the flow tube; such a variation of the addition sites is an elegant way of adding the reactants as a function of residence time.

In step a) of the process according to the invention, there is no need of separately feeding the CO₂, the alkene and the alkoxide to the reaction.

In step b) the α,β-ethylenically unsaturated carboxylic acid salt is allowed to precipitate out from the crude reaction product. The ability of the α,β-ethylenically unsaturated carboxylic acid salt to precipitate out from the crude reaction product is primarily dependent on the temperature of the crude reaction product and the choice of the organic solvent.

As described above, the carboxylic acid salt is preferably insoluble in the organic solvent within the temperature ranges of the process steps, especially steps a) to c). In this case, the α,β-ethylenically unsaturated carboxylic acid salt precipitates out from the crude reaction product immediately upon formation and is maintained in solid form.

The choice of pressure and especially temperature at which step b) is performed is generally dependent on the properties of the α,β-ethylenically unsaturated carboxylic acid salt. With regard to the efficiency of the process, the immediate precipitation of the carboxylic acid salt from the crude reaction product upon formation, and thus at the same temperature and pressure at which step a) is performed, is preferable.

In another embodiment, the α,β-ethylenically unsaturated carboxylic acid salt is allowed to precipitate out from the crude reaction product at a lower temperature and similar pressure to the one at which step a) is performed.

Step b) of the process according to the invention may be performed continuously or discontinuously. Step b) is preferably performed continuously.

In step c) of the process, at least part of the crude reaction product is subjected to a mechanical separation step while maintaining the alcohol by-product in liquid form. A solid phase comprising the α,β-ethylenically unsaturated carboxylic acid salt is obtained along with a liquid phase comprising the carboxylation catalyst, the organic solvent and the alcohol by-product.

In order to be able to recover the precipitated α,β-ethylenically unsaturated carboxylic acid salt as a solid phase of high purity it is essential to maintain the alcohol by-product in a liquid or dissolved form. The temperature of the crude reaction product during the mechanical separation step c), along with the choice of organic solvent, may be crucial for maintaining the alcohol by-product in liquid form while maintaining the carboxylic acid salt in solid form. The optimal temperature range for performing step c) of the process depends on the properties of the obtained alcohol by-product, and thus on the alkoxide used. This is especially critical if the alcohol by-product is non-soluble in the organic solvent at, e.g., low temperatures.

Generally, step c) may be performed at a temperature in the range of 0 to 150 °C.

In one embodiment, the temperature at which step c) is performed is the same as the temperature at which step a) is performed.

In another embodiment, the temperature at which step c) is performed is lower than the temperature at which step a) is performed. For example, if the alkoxide is sodium tert-butoxide and the alkene is ethene, the obtained carboxylic acid salt is sodium acrylate and the obtained alcohol by-product is tert-butanol. Tert-butanol has a melting point of approximately 25 °C at 1 bar. It is crucial to perform the mechanical separation step c) at a temperature well above this temperature so as to keep tert-butanol in its liquid form and allow for a high degree of purity of the solid phase comprising the carboxylic acid salt. In this embodiment, it is preferable that step a) is performed at a temperature in the range of 130 to 160 °C, while step c) is performed at a temperature in the range of 70 to 90 °C.

Step c) may be performed at the same or lower total pressure than step a). Step c) is preferably performed at lower total pressure than step a). The crude reaction product obtained from step a) may, for example, be decompressed into a decompression vessel before it is directed into step c). Any gas released from the crude reaction product during decompression, i.e. CO₂ and optionally alkene, is preferably recycled into the process, in particular into step a). Step c) is preferably performed at a total pressure in the range of 0.01 to 20 bara, for example 0.1 to 10 bar.

Suitably, the solid phase obtained in step c) comprises essentially all of the precipitated α,β-ethylenically unsaturated carboxylic acid salt of the crude reaction product subjected to the mechanical separation step c). For example, the solid phase comprises at least 95 wt.-%, at least 97 wt.-%, at least 99 wt.-%, or at least 99.5 wt.-% of the precipitated α,β-ethylenically unsaturated carboxylic acid salt of the crude reaction product subjected to the mechanical separation step c).

Preferably, the solid phase obtained in step c) essentially comprises only the α,β-ethylenically unsaturated carboxylic acid salt. For example, the carboxylic acid salt makes up at least 95 wt.-%, at least 97 wt.-%, at least 99 wt.-%, or at least 99.5 wt.-% of the solid phase obtained in step c), based on the total weight of the solid phase.

Suitably, the liquid phase obtained in step c) comprises essentially all of the alcohol by-product of the crude reaction product subjected to the mechanical separation step c). Further to the alcohol by-product, the liquid phase obtained in step c) generally comprises organic solvent and carboxylation catalyst, as well as unreacted alkene, carbon dioxide and alkoxide.

The mechanical separation step c) may be carried out by any technique suitable for the separation of a solid phase from a liquid phase. Examples of mechanical separation steps include a filtration step, for example via vacuum filters or pressure filters; a centrifugation step, for example via filtering or sedimenting centrifuges or hydrocyclones; or a sedimentation step.

Preferably, the mechanical separation step c) comprises a filtration step.

Step c) of the process according to the invention may be performed continuously or discontinuously. Step c) is preferably performed continuously, for example by use of a belt filter. A suitable belt filter comprises a gravity drainage zone, a linear compression pressure zone and a roller compression zone. The crude reaction product is first fed to the flat or inclined gravity drainage zone, where gravity drainage of the liquid phase takes place. Next, the feed is passed through the linear compression pressure zone comprising an upper belt and a lower belt arranged so as to sandwich the feed together at low pressure. Finally, the feed is passed through a roller compression zone wherein the feed, still sandwiched between the upper and lower belt, is subjected to high pressure by means of a system of rollers.

In preferred embodiments, the process may comprise a step d), wherein the alcohol by-product and optionally the organic solvent is distilled off from the liquid phase obtained in step c). An alcohol by-product distillate fraction and optionally a solvent distillate fraction, and a residue fraction are obtained. The relative boiling points of the alcohol by-product and the organic solvent determine whether the alcohol by-product or the organic solvent is first distilled off from the liquid phase.

When the boiling point of the organic solvent is higher than the boiling point of the alcohol by-product, it is preferable that only the alcohol by-product is distilled off from the liquid phase obtained in step c). In this case, the obtained alcohol by-product distillate fraction comprises most of the alcohol by-product, preferably at least 90%, more preferably at least 95%, in particular at least 97%, most preferably at least 99% of the alcohol by-product comprised in the liquid phase fed into step d). The residue fraction generally comprises organic solvent and carboxylation catalyst, as well as unreacted alkene, carbon dioxide and alkoxide.

When the boiling point of the organic solvent is lower than the boiling point of the alcohol by-product, it is preferable that both the alcohol by-product and the organic solvent are distilled off from the liquid phase obtained in step c). In this case, the obtained solvent distillate fraction comprises most of the organic solvent, preferably at least 90%, more preferably at least 95%, in particular at least 97%, most preferably at least 99% of the organic solvent comprised in the liquid phase fed into step d). The obtained alcohol by-product distillate fraction comprises most of the alcohol by-product, preferably at least 85%, more preferably at least 90%, in particular at least 95% of the alcohol by-product comprised in the liquid phase fed into step d). The alcohol by-product distillate fraction and the solvent distillate fraction may be present as a single fraction or separate fractions. The residue fraction generally comprises carboxylation catalyst, as well as unreacted alkene, carbon dioxide and alkoxide, and suitably a small amount of alcohol by-product to allow for the carboxylation catalyst to be easily recycled to step a).

Step d) may, for example, be carried out in a distillation unit comprising a distillation column which may comprise trays or packing material. The liquid phase obtained in step c) is fed into the distillation unit, where it is heated and/or exposed to reduced pressure.

The pressure in step d) is, for example, in the range of 0.0001 to 10 bar, preferably 0.001 to 5 bar, most preferably 0.01 to 2 bar. The temperature in the bottom of the distillation column is preferably kept well above the boiling temperature of the alcohol by-product at the distillation pressure. The temperature in the bottom of the distillation column is, for example, in the range of 60 to 200 °C, preferably 80 to 180 °C.

Step d) may be performed continuously or discontinuously. It is preferably performed continuously.

In a preferred embodiment, at least part of the obtained residue fraction is recycled to step a). The residue fraction may be recycled to step a) with or without further workup steps; preferably without further workup steps. Further workup steps may include drying.

In some embodiments, the catalyst system may have low solubility in the alcohol by-product. In these cases, it is preferable that the boiling point of the organic solvent is higher than that of the alcohol by-product, so that the alcohol by-product is distilled off first from the liquid phase in step d) and the residue fraction comprises the organic solvent. Thus, the catalyst may conveniently be recycled to step a) in the organic solvent. When the boiling point of the alcohol by-product is relatively high, it may accordingly be useful to employ a higher-boiling organic cosolvent or a solvent mixture comprising at least one solvent component having a higher boiling point than the alcohol by-product. Suitable higher-boiling organic cosolvents that can be used individually or in admixture include the above-mentioned polyalkylene glycols or di- or mono(C₁-C₄-alkyl ether) polyalkylene glycols.

In preferred embodiments, the process may comprise a step e), wherein at least part of the alcohol by-product distillate fraction obtained in step d) is contacted with an alkaline material to obtain a regenerated alkoxide. In this embodiment, the alkoxide is regenerated by reacting the alcohol by-product with an alkaline material which is capable of deprotonating the alcohol by-product such that the alkoxide is regenerated.

The alcohol by-product is comprised in the alcohol by-product distillate fraction obtained in the distillation step d); generally outside of the carboxylation reactor. It can therefore be contacted with the alkaline material outside of the carboxylation reactor, i.e. at low carbon dioxide partial pressure. Nucleophilic alkaline materials that are inactivated under the conditions of the reaction between alkene and carbon dioxide, i.e. at high carbon dioxide partial pressure, may thus be used for regenerating the alkoxide. This is advantageous as some of these alkaline materials, for example, sodium hydroxide, are less costly than other less nucleophilic alkaline materials. It is preferable that the alcohol by-product distillate fraction is discrete from the solvent distillate fraction optionally obtained in step d).

The alkaline material used in step e) is preferably selected from alkali metals or alkaline earth metals, alkali metal and alkaline earth metal oxides and alkali metal and alkaline earth metal hydroxides and their mixtures, in particular from Li, Na, K, Ca, Li₂O, Na₂O, K₂O, CaO, LiOH, NaOH, KOH, Ca(OH)₂, and their mixtures. Sodium hydroxide is the most preferred alkaline material used in step e).

Alternative alkaline materials that can be used in step e) are, for example, selected from alkali metal or alkaline earth metal hydrides, amides, phosphides, silanolates, lower alkoxide (e.g. methoxide), alkyls, and aryls. These are similarly or even more reactive towards the alcohol by-product but more difficult to handle and/or more costly than the aforementioned preferred alkaline materials.

Suitable alkali metal or alkaline earth metal hydrides are, for example, lithium hydride, sodium hydride, potassium hydride, magnesium hydride, and calcium hydride.

Suitable lower alkoxides are, for example, lithium methoxide, lithium ethoxide, sodium methoxide, sodium ethoxide, sodium isopropoxide, potassium methoxide, magnesium methoxide, and calcium methoxide.

Suitable alkali metal or alkaline earth metal amides are, for example, LiNH₂, NaNH₂, KNH₂, LiNMe₂, LiNEt₂, LiN(iPr)₂, NaNMe₂, NaNEt₂, NaN(iPr)₂, KNMe₂, KNEt₂, KN(iPr)₂, (Me = Methyl; Et = Ethyl; iPr = Isopropyl). The suitable amides also include silicon-containing amides such as sodium hexamethyldisilazide (NaHMDS), potassium hexamethyldisilazide (KHMDS) or lithium hexamethyldisilazide (LiHMDS).

Suitable alkali metal or alkaline earth metal phosphides are, for example, those of the formula M²PR¹⁰¹₂ in which M² is an alkali metal or an equivalent of an alkaline earth metal, and R¹⁰¹ is C₁₋₁₂-alkyl or C₆₋₁₀-aryl, for example KPPh₂ or NaPPh₂ (Ph =Phenyl).

Suitable alkali metal or alkaline earth metal silanolates are, for example, those of the formula M²OSi(C₁₋₄-Alkyl)₃ in which M² is an alkali metal or an equivalent of an alkaline earth metal, for example NaOSiMe₃.

Suitable alkali metal or alkaline earth metal alkyls or aryls are, for example, lithium alkyl and lithium aryl compounds, such as methyllithium, n-butyllithium, sec-butyllithium, tert-butyllithium, phenyllithium, where the benzene ring may bear substituents at any position (e.g. OCH₃, CH₂NMe₂, CONR₂), cyclohexyllithium, where the cyclohexyl ring may comprise heteroatoms (e.g. O, N, S), ethyllithium, lithium pentadienyl, lithium 2-furanyl, lithium 2-thiophenyl, lithium ethynyl. Also suitable are sodium alkyl and sodium aryl compounds, such as sodium cyclopentadienyl.

The suitable alkaline earth metal alkyls and aryls include magnesium alkyl and magnesium aryl compounds (Grignard reagents) of the general formula R¹⁰²MgX, wherein R¹⁰² may be one of the alkyl and aryl residues listed above for the lithium alkyl and lithium aryl compounds and X may be F, Cl, Br or I.

When the alkaline material is selected from alkali or alkaline earth metals, such as sodium, lithium, or potassium, in particular sodium, the deprotonation of the alcohol by-product is coupled with a redox reaction. The alkali or alkaline earth metal is oxidized to the alkali metal or alkaline earth metal cation and the oxygen-bound proton of the alcohol by-product is reduced to hydrogen.

The regeneration of the alkoxide is preferably performed in the liquid or supercritical phase at pressures in the range of 0.0001 to 150 bar, preferably 0.001 to 100 bar, more preferably 0.001 to 60 bar. The temperature may, for example, be in the range of -20 to 300 °C, preferably 20 to 250 °C, more preferably 40 to 200 °C.

Step e) may be performed continuously or discontinuously. It is preferably performed continuously.

Preferably, at least part of the regenerated alkoxide obtained in step e) is recycled to step a). The regenerated alkoxide obtained in step e) may, for example, be dried and recycled to step a) in the form of a solid or in the form of a solution in the organic solvent.

In preferred embodiments, the process comprises a step f) wherein the solid phase obtained in step c) is contacted with a wash liquid to obtain a used wash liquid and a purified solid phase in which the carboxylic acid salt is enriched. Conveniently, the wash liquid is suitable for removing significant amounts of carboxylation catalyst impurities from the solid phase, thus allowing the carboxylic acid salt to be obtained in a higher degree of purity.

The wash liquid is suitably selected from liquids which are inert with regard to the carboxylic acid salt and in which the carboxylic acid salt is essentially non-soluble. The wash liquid may be selected from alkanes, such as butane, pentane, hexane or heptane in their linear or branched form, for example n-pentane; alcohols, such as propanol, butanol, pentanol or hexanol in their linear or branched form, and ethers, such as diethyl ether, methyl tert-butyl ether and tetrahydrofuran. In a preferred embodiment, the wash liquid is selected from n-pentane and the conjugate alcohol of the alkoxide. Most preferably, the wash liquid is the conjugate alcohol of the alkoxide.

In one embodiment, the wash liquid is the conjugate alcohol of the alkoxide, i.e. the alcohol by-product. In this embodiment, it is preferable that at least part of the used wash liquid is fed into step d). For example, at least 40%, such as at least 60% or at least 80% of the used wash liquid is recycled to step d).

In another embodiment, the wash liquid is the organic solvent of step a). In this case, it is preferable that at least part of the used wash liquid is recycled to step a). For example, at least 40%, such as at least 60% or at least 80% of the used wash liquid is recycled to step a). The organic solvent to be used as wash liquid is favorably purified prior to being recycled to step a). For example, the organic solvent to be used as wash liquid may be distilled prior to being recycled to step a).

It may be necessary to heat the wash liquid above its melting point prior to use. For example, tert-butanol has a melting point of approximately 25 °C at 1 bar absolute. Tert-butanol is thus preferably heated to a temperature of at least 40 °C, more preferably at least 60 °C, and most preferably at least 70 °C, for example at least 80 °C.

Step f) of the process according to the invention may be performed continuously or discontinuously. Step f) is preferably performed continuously. When a filter is used in the mechanical separation step c), step f) may be performed directly on the filter. Alternatively, step f) may be carried out by suspending the solid phase obtained in step c) in the wash liquid and subsequently carrying out a mechanical separation step, such as a filtration.

The invention is described in more detail by the following examples, which illustrate the production of sodium acrylate.

High performance liquid chromatography (HPLC) analysis of the reaction products after treatment with a 0.1% aqueous H₃PO₄ solution was used to analytically confirm the identity and purity of generated sodium acrylate. It is considered that the turnover numbers (TONs) reported below are subject to errors of up to 15% originating from the experimental setup. This is due to inherent weighing errors and the sticky, viscous nature of the autoclave output

In the examples, the following abbreviations are used:
- dcpe:: 1,2-bis(dicyclohexylphosphino)ethane
- DMF:: N,N-dimethylformamide
- TON:: turnover number with respect to transition metal

### Example 1

A 270 mL steel autoclave was charged with Pd(PPh₃)₄ (0.400 mmol, 0.463 g), dcpe (0.440 mmol, 0.187 g), sodium tert-butoxide (40.00 mmol, 3.84 g) and DMF (60 mL) using standard Schlenk techniques under an atmosphere of argon. The autoclave was stirred at 500 rpm for 15 min to solubilize the reactants. The autoclave was pressurized with CO₂ (35 bar), ethene (10 bar) and nitrogen (25 bar) at 25 °C (total pressure of 70 bar). After stirring at 145 °C for 16 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via warm filtration at 80 °C under reduced pressure. The obtained sticky solid was washed with 3 × 25 mL of DMF, followed by 3 × 25 mL of n-pentane. The solid residue was dried under vacuum to give the product as a white solid. A quantitative yield of 3.90 g was obtained, corresponding to a TON of 104.

### Example 2

A 270 mL steel autoclave was charged with Pd(PPh₃)₄ (0.400 mmol, 0.463 g), dcpe (0.440 mmol, 0.187 g), sodium 3,7-dimethyloctan-3-olate (40.00 mmol, 7.21 g) and DMF (60 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A yield of 3.00 g (80%) was obtained, corresponding to a TON of 80.

### Example 3

A 270 mL steel autoclave was charged with Pd(PPh₃)₄ (0.400 mmol, 0.463 g), dcpe (0.440 mmol, 0.187 g), sodium 3,7-dimethyloctan-3-olate as a 48% solution in 3,7-dimethyloctan-3-ol (40 mmol, 15.4 g) and DMF (53 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A yield of 2.88 g (77%) was obtained, corresponding to a TON of 77.

### Example 4

A 270 mL steel autoclave was charged with Pd(PPh₃)₄ (0.400 mmol, 0.463 g), dcpe (0.440 mmol, 0.187 g), sodium 3,7-dimethyloctan-3-olate as a 48% solution in 3,7-dimethyloctan-3-ol (40.00 mmol, 15.4 g), 3,7-dimethyloctan-3-ol (20 mL) and DMF (30 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A yield of 3.05 g (81%) was obtained, corresponding to a TON of 81.

### Example 5

A 270 mL steel autoclave was charged with Pd(PPh₃)₄ (0.400 mmol, 0.463 g), dcpe (0.440 mmol, 0.187 g), sodium 3,7-dimethyloctan-3-olate as a 48% solution in 3,7-dimethyloctan-3-ol (40.00 mmol, 15.4 g), 3,7-dimethyloctan-3-ol (20 mL) and DMF (30 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A yield of 2.84 g (76%) was obtained, corresponding to a TON of 76.

### Example 6

A 270 mL steel autoclave was charged with Pd(PPh₃)₄ (0.400 mmol, 0.463 g), dcpe (0.440 mmol, 0.187 g), sodium 3-methylpentan-3-olate (40.00 mmol, 4.97 g) and DMF (60 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A quantitative yield of 3.91 g, was obtained, corresponding to a TON of 104.

### Example 7

A 270 mL steel autoclave was charged with Pd(PPh₃)₄ (0.400 mmol, 0.463 g), dcpe (0.440 mmol, 0.187 g), sodium 3-methylpentan-3-olate (40.00 mmol, 4.97 g) and DMF (60 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A yield of 3.14 g (84%) was obtained, corresponding to a TON of 84.

### Example 8

A 270 mL steel autoclave was charged with Pd(PPh₃)₄ (0.400 mmol, 0.463 g), dcpe (0.440 mmol, 0.187 g), sodium 3,7-dimethyloctan-3-olate as a 51% solution in 3,7-dimethyloctan-3-ol (40.00 mmol, 14.1 g), and DMF (54 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A quantitative yield of 4.28 g was obtained, corresponding to a TON of 114.

### Example 9

A 270 mL steel autoclave was charged with Pd(PPh₃)₄ (0.400 mmol, 0.463 g), dcpe (0.440 mmol, 0.187 g), sodium 3-ethyl-pentan-3-olate (40.00 mmol, 5.52 g) and DMF (60 mL) using standard Schlenk techniques under an atmosphere of argon. The autoclave was stirred at 500 rpm for 15 min to solubilize the reactants. The autoclave was pressurized with CO₂ (35 bar) and ethene (10 bar) at 25 °C. After stirring at 145 °C for 5 h at 750 rpm, the autoclave was cooled to 80 °C, the pressure was released and the reaction mixture, i.e. crude reaction product, was transferred into a 100 mL bottle. The residues in the autoclave vessel were recovered with 15 mL of DMF and transferred into the bottle. The product was separated from the reaction mixture via filtration at room temperature under reduced pressure. The obtained sticky solid was washed with 3 × 25 mL of DMF, followed by 3 × 25 mL of n-pentane. The solid residue was dried under vacuum to give the product as a white solid. A quantitative yield of 3.92 g was obtained, corresponding to a TON of 104.

## Claims

1. A catalytic process for preparing an α,β-ethylenically unsaturated carboxylic acid salt, comprising
a) contacting an alkene and carbon dioxide with a carboxylation catalyst, an organic solvent, wherein the organic solvent is N,N-dimethylformamide, and an alkoxide having a secondary or tertiary carbon atom directly bound to an [O⁻] group, to obtain a crude reaction product comprising the α,β-ethylenically unsaturated carboxylic acid salt and an alcohol by-product which is the conjugate acid of the alkoxide,
b) allowing the α,β-ethylenically unsaturated carboxylic acid salt to precipitate out from the crude reaction product; and
c) subjecting at least part of the crude reaction product to a mechanical separation step while maintaining the alcohol by-product in liquid form to obtain a solid phase comprising the α,β-ethylenically unsaturated carboxylic acid salt and a liquid phase comprising the carboxylation catalyst, the organic solvent and the alcohol by-product.

2. The catalytic process according to claim 1, wherein the mechanical separation step c) comprises a filtration step.

3. The catalytic process according to claim 1 or 2, additionally comprising
d) distilling the alcohol by-product and optionally the organic solvent off from the liquid phase to obtain an alcohol by-product distillate fraction and optionally a solvent distillate fraction, and a residue fraction.

4. The catalytic process according to claim 3, additionally comprising recycling at least part of the residue fraction to step a).

5. The catalytic process according to claim 3 or 4, additionally comprising
e) contacting at least part of the alcohol by-product distillate fraction obtained in step d) with an alkaline material to regenerate an alkoxide.

6. The catalytic process according to claim 5, additionally comprising recycling at least part of the regenerated alkoxide to step a).

7. The catalytic process according to any of the preceding claims, additionally comprising
f) contacting the solid phase obtained in step c) with a wash liquid to obtain a spent wash liquid and a purified solid phase in which the carboxylic acid salt is enriched.

8. The catalytic process according to claim 7, wherein the wash liquid is selected from alkanes and alcohols.

9. The catalytic process according to claim 8, wherein the wash liquid is the alcohol by-product.

10. The catalytic process according to claim 8, wherein the wash liquid is the organic solvent.

11. The catalytic process according to any of the preceding claims, wherein step c) is performed at a temperature in the range of 0 to 150 °C.

12. The catalytic process according to any of the preceding claims, wherein the alkoxide is a sodium alkoxide, preferably a sodium alkoxide having a tertiary carbon atom directly bound to an [O⁻] group.

13. The catalytic process according to any of the preceding claims, wherein the alkene is ethene and the α,β-ethylenically unsaturated carboxylic acid salt is sodium acrylate.

14. The catalytic process according to any of the preceding claims, wherein the carboxylation catalyst is a nickel or palladium complex which comprises a bidentate P,X ligand in which X is selected from the group consisting of P, N, O, and carbene, and the P and X atoms are separated by a bivalent linker that comprises 2 to 4 bridging atoms.

## Patentansprüche

1. Katalytisches Verfahren zur Herstellung eines Salzes einer α,β-ethylenisch ungesättigten Carbonsäure, bei dem man
a) ein Alken und Kohlendioxid mit einem Carboxylierungskatalysator, einem organischen Lösungsmittel, wobei es sich bei dem organischen Lösungsmittel um N,N-Dimethylformamid handelt, und einem Alkoxid mit einem direkt an eine [O⁻]-Gruppe gebundenen sekundären oder tertiären Kohlenstoffatom in Kontakt bringt, wobei man ein rohes Reaktionsprodukt erhält, das das Salz einer α,β-ethylenisch ungesättigten Carbonsäure und ein Alkohol-Nebenprodukt, bei dem es sich um die konjugierte Säure des Alkoxids handelt, umfasst,
b) das Salz einer α,β-ethylenisch ungesättigten Carbonsäure aus dem rohen Reaktionsprodukt ausfallen lässt und
c) mindestens einen Teil des rohen Reaktionsprodukts einem mechanischen Trennschritt unterwirft, wobei das Alkohol-Nebenprodukt in flüssiger Form gehalten wird, wobei man eine feste Phase, die das Salz einer α,β-ethylenisch ungesättigten Carbonsäure umfasst, und eine flüssige Phase, die den Carboxylierungskatalysator, das organische Lösungsmittel und das Alkohol-Nebenprodukt umfasst, erhält.

2. Katalytisches Verfahren nach Anspruch 1, bei dem der mechanische Trennschritt c) einen Filtrationsschritt umfasst.

3. Katalytisches Verfahren nach Anspruch 1 oder 2, bei dem man zusätzlich
d) das Alkohol-Nebenprodukt und gegebenenfalls das organische Lösungsmittel aus der flüssigen Phase abdestilliert, wobei man eine Alkohol-Nebenprodukt-Destillatfraktion und gegebenenfalls eine Lösungsmittel-Destillatfraktion und eine Rückstandsfraktion erhält.

4. Katalytisches Verfahren nach Anspruch 3, bei dem man zusätzlich mindestens einen Teil der Rückstandsfraktion zu Schritt a) zurückführt.

5. Katalytisches Verfahren nach Anspruch 3 oder 4, bei dem man zusätzlich
e) mindestens einen Teil der in Schritt d) erhaltenen Alkohol-Nebenprodukt-Destillatfraktion zur Regeneration eines Alkoxids mit einer alkalischen Substanz in Kontakt bringt.

6. Katalytisches Verfahren nach Anspruch 5, bei dem man zusätzlich zumindest einen Teil des regenerierten Alkoxids zu Schritt a) zurückführt.

7. Katalytisches Verfahren nach einem der vorhergehenden Ansprüche, bei dem man zusätzlich
f) die in Schritt c) erhaltene feste Phase mit einer Waschflüssigkeit in Kontakt bringt, wobei man eine gebrauchte Waschflüssigkeit und eine gereinigte feste Phase, in der das Carbonsäuresalz angereichert ist, erhält.

8. Katalytisches Verfahren nach Anspruch 7, bei dem man die Waschflüssigkeit aus Alkanen und Alkoholen auswählt.

9. Katalytisches Verfahren nach Anspruch 8, bei dem es sich bei der Waschflüssigkeit um das Alkohol-Nebenprodukt handelt.

10. Katalytisches Verfahren nach Anspruch 8, bei dem es sich bei der Waschflüssigkeit um das organische Lösungsmittel handelt.

11. Katalytisches Verfahren nach einem der vorhergehenden Ansprüche, bei den man Schritt c) bei einer Temperatur im Bereich von 0 bis 150 °C durchführt.

12. Katalytisches Verfahren nach einem der vorhergehenden Ansprüche, bei dem es sich bei dem Alkoxid um ein Natriumalkoxid, vorzugsweise ein Natriumalkoxid mit einem direkt an eine [O⁻]-Gruppe gebundenen tertiären Kohlenstoffatom, handelt.

13. Katalytisches Verfahren nach einem der vorhergehenden Ansprüche, bei dem es sich bei dem Alken um Ethen handelt und es sich bei dem Salz einer α,β-ethylenisch ungesättigten Carbonsäure um Natriumacrylat handelt.

14. Katalytisches Verfahren nach einem der vorhergehenden Ansprüche, bei dem es sich bei dem Carboxylierungskatalysator um einen Nickel- oder Palladiumkomplex handelt, der einen zweizähnigen P,X-Liganden umfasst, wobei X aus der Gruppe bestehend aus P, N, O und Carben ausgewählt ist und das P- und X-Atom durch einen zweiwertigen Linker mit 2 bis 4 Brückenatomen getrennt sind.

## Revendications

1. Procédé catalytique de préparation d'un sel d'acide carboxylique α,β-éthyléniquement insaturé, comprenant
a) la mise en contact d'un alcène et de dioxyde de carbone avec un catalyseur de carboxylation, un solvant organique, le solvant organique étant le N,N-diméthylformamide, et un alcoxyde ayant un atome de carbone secondaire ou tertiaire directement lié à un groupe [O⁻], pour obtenir un produit de réaction brut comprenant le sel d'acide carboxylique α,β-éthyléniquement insaturé et un sous-produit de type alcool qui est l'acide conjugué de l'alcoxyde,
b) le fait de laisser le sel d'acide carboxylique α,β-éthyléniquement insaturé précipiter le produit de réaction brut ; et
c) la soumission d'au moins une partie du produit de réaction brut à une étape de séparation mécanique tout en maintenant le sous-produit de type alcool sous forme liquide pour obtenir une phase solide comprenant le sel d'acide carboxylique α,β-éthyléniquement insaturé et une phase liquide comprenant le catalyseur de carboxylation, le solvant organique et le sous-produit de type alcool.

2. Procédé catalytique selon la revendication 1, dans lequel l'étape de séparation mécanique c) comprend une étape de filtration.

3. Procédé catalytique selon la revendication 1 ou 2, comprenant en outre
d) la distillation du sous-produit de type alcool et éventuellement du solvant organique de la phase liquide afin d'obtenir une fraction de distillat de sous-produit de type alcool et éventuellement une fraction de distillat de solvant, et une fraction de résidu.

4. Procédé catalytique selon la revendication 3, comprenant en outre le recyclage d'au moins une partie de la fraction de résidu à l'étape a).

5. Procédé catalytique selon la revendication 3 ou 4, comprenant en outre
e) la mise en contact d'au moins une partie de la fraction de distillat de sous-produit de type alcool obtenue à l'étape d) avec un matériau alcalin pour régénérer un alcoxyde.

6. Procédé catalytique selon la revendication 5, comprenant en outre le recyclage d'au moins une partie de l'alcoxyde régénéré à l'étape a).

7. Procédé catalytique selon l'une quelconque des revendications précédentes, comprenant en outre
f) la mise en contact de la phase solide obtenue à l'étape c) avec un liquide de lavage pour obtenir un liquide de lavage utilisé et une phase solide purifiée dans laquelle le sel d'acide carboxylique est enrichi.

8. Procédé catalytique selon la revendication 7, dans lequel le liquide de lavage est sélectionné parmi des alcanes et des alcools.

9. Procédé catalytique selon la revendication 8, dans lequel le liquide de lavage est le sous-produit de type alcool.

10. Procédé catalytique selon la revendication 8, dans lequel le liquide de lavage est le solvant organique.

11. Procédé selon l'une quelconque des revendications précédentes, dans lequel l'étape c) est réalisée à une température dans la plage de 0 à 150 °C.

12. Procédé catalytique selon l'une des revendications précédentes, dans lequel l'alcoxyde est un alcoxyde de sodium, de préférence un alcoxyde de sodium ayant un atome de carbone tertiaire directement lié à un groupe [O⁻].

13. Procédé catalytique selon l'une des revendications précédentes, dans lequel l'alcène est l'éthène et le sel d'acide carboxylique α,β-éthyléniquement insaturé est l'acrylate de sodium.

14. Procédé catalytique selon l'une quelconque des revendications précédentes, dans lequel le catalyseur de carboxylation est un complexe de nickel ou de palladium qui comprend un ligand bidentate P,X dans lequel X est sélectionné dans le groupe constitué par P, N, O et carbène, et les atomes P et X sont séparés par un lieur bivalent composé de 2 à 4 atomes pontants.
